# EUROPEAN PATENT APPLICATION

(11) **EP 1 182 250 A2**
(43) Date of publication of application: **27.02.2002**
(21) Application number: 00120573.1
(22) Date of filing: 20.09.2000
(51) Int. Cl.: C12M 3/00

(54) **Apparatus, systems and methods for microinjection of samples into amphibian oocytes**

(30) Priority: 25.08.2000 JP 2000256381
(71) Applicant: Hitachi, Ltd., Chiyoda-ku, Tokyo 101-8010 (JP)
(72) Inventor: Takeshita, Tomoko, Hitachi, Ltd., Int. Prop. Group, Chiyoda-ku, Tokyo 100-8220 (JP); Otomo, Jun, Hitachi, Ltd., Intellect. Prop. Group, Chiyoda-ku, Tokyo 100-8220 (JP); Nomura, Sayuri, Hitachi, Ltd., Intell. Prop. Group, Chiyoda-ku, Tokyo 100-8220 (JP); Matsunami, Shokichi, Hitachi Ltd., Int. Pro. Group, Chiyoda-ku, Tokyo 100-8220 (JP); Moriya, Noboru, Hitachi Ltd., Intell. Prop. Group, Chiyoda-ku, Tokyo 100-8220 (JP); Saitou, Sakae, Hitachi Ltd., Intell. Prop. Group, Chiyoda-ku, Tokyo 100-8220 (JP)
(74) Representative: Beetz & Partner Patentanwälte

(57) **Abstract**

The invention provides an apparatus for the microinjection of samples into amphibian oocytes, comprising a tray (9) for holding a plurality of amphibian oocytes (13), an injection needle (6) for injecting a sample into the amphibian oocytes (13), driving means (4, 11, 12) for moving the relative position between the tray (9) and the injection needle (6) and controlling means (1, 2) for controlling the movement by setting the depth of the injection needle (6) relative to the tray (9) or the amphibian oocytes (13) in the sample injection and for injecting the sample into the amphibian oocytes (13) at that depth.

According to the present invention, the sample can be injected into amphibian oocytes (13) with constant depth precisely, and the quality of oocytes or the positional site of needle injection can be recorded as product information.

## Description

### Background of the Invention

### (1) Technical Field

The present invention relates to an automated apparatus for injecting a sample of gene, pigment, protein, peptide or drug into oocytes of amphibia such as frogs using a pipette-like needle. The present invention further pertains to a method for injecting samples of genes, pigments, proteins, peptides or drugs into a specified position of the amphibian oocytes, the amphibian oocytes with a guaranteed quality regarding the injection of the sample, and a method for selling or assigning the amphibian oocytes into which the sample has been injected at a specified position and depth.

### (2) Background Art

Since oocytes of frogs such as Xenopus laevis, into which a gene, pigment, protein, peptide or drug is injected, have a comparatively large size and can be obtained at low cost and in large quantities, they are widely used for the purpose of confirming actions of pigments, proteins, peptides and drugs on viable cells, analysis of gene functions, and production of proteins as gene products. To this purpose, research scientists breed frogs and collect the oocytes.

Heretofore, in the injection of a sample such as gene, pigment, protein, peptide and drug into the oocytes of amphibia such as frogs, technicians manually injected with a pipette-like needle in which these samples are packed, into the oocytes under microscopic observation using a manipulator. The pipette is mounted on the injector, and a constant amount of sample is injected and released into the cells by the action of an oil pressure or an air pressure. Further, JP-A-5-192171 and JP-A-6-343478 disclose a method for injection and release of a constant amount of sample into cells by applying a voltage. The above prior art discloses techniques for injection of samples by approaching a needle manually to the cell with microscopic observation of the cell.

### Summary of the Invention

### (1) Problems to be solved by the invention

The above manual injection of samples such as genes involves the problem that the rate of oocytes, into which the sample was successfully injected varies depending on the skills and experiences of technicians. This is due to a difference in the number of oocytes into which the sample could be injected within a fixed time, depending on the individual technician, and as a result, the degree of denaturation of the sample varies in a time-dependent manner among the technicians. It is underlying problem of the present invention to provide a method to give a constant number of cells per hour to be treated without skills and experiences of technicians.

Further, the above described prior art did not consider to unify the injection depth of samples into the oocytes at a constant depth. Consequently, it was difficult to control the depth even by skilled technicians. As a result, the injection of a sample into a specified cell organelle such as a nucleus had to depend on chance. It is another problem of the present invention to provide an easier method of injecting a sample into a cellular organelle such as a nucleus, in which the injection can be controlled in the depth direction by unifying the injection depth of the sample.

Furthermore, in the above described prior art, since no consideration was given for memorizing information of cells in the injection of a sample, it was difficult to obtain a correlation between the information on the sample injection and the subsequent cell reaction. Consequently, it is a further problem of the present invention to provide a method for obtaining the correlation between them.

In addition, in the conventional techniques, the oocytes were prepared individually, and a mass production or a production on demand in good timing were impossible.

Consequently, it is a further problem of the present invention to provide the oocytes, into which a specified sample has been injected, or the production, sale and transportation of the oocytes with a guaranteed injection of a specified sample at a constant position.

### (2) Means for solving the problems

The above problems are solved by the independent claims. The dependent claims relate to preferred embodiments of the concept of the present invention.

In order to solve the above problems, the present invention provides an apparatus for automatically injecting a sample such as a gene, pigment, protein, peptide or drug into oocytes of amphibia such as frogs at any constant position and at any constant depth of the oocytes by using a pipette-like needle.

Namely, the present invention provides an apparatus for microinjection of samples into amphibian oocytes comprising a tray for holding a plurality of the amphibian oocytes, an injection needle for injecting a sample into the amphibian oocytes, driving means for moving the relative position of the tray to the injection needle and controlling means for controlling the movement by putting in the depth of the injection needle for the tray or the amphibian oocytes in the injection of the sample, and injecting the sample into the amphibian oocytes at that depth.

The present invention further provides a system for microinjection of samples into amphibian oocytes comprising a tray for holding a plurality of the amphibian oocytes, an injection needle for injecting a sample into the amphibian oocytes, driving means for moving the relative position of the tray to the injection needle in a three-dimensional direction, controlling means for controlling the movement, information obtaining means for obtaining a visual information of the amphibian oocytes in the microinjection, and memorizing means for accumulating the information, and injecting the sample into the amphibian oocytes.

As a result, the sample can be injected rapidly into a plurality of amphibian oocytes at an almost constant depth.

Further, the tray has a plurality of wells having a cylindrical structure with a flat base or with a conical base having a maximum diameter of 105 - 150 % of the diameter of the amphibian oocytes. As a result, the sample can be injected into the identical surface position in about 80 % of the oocytes on the tray without applying any special means.

We have found that when mRNA was injected into the oocytes, in case that the mRNA was injected into the animal hemisphere of the oocyte, or mRNA was injected into the vegetal hemisphere, the expression efficiency or the functional expression efficiency is different. Namely, in order to suppress a variation of the expression efficiency of the function of a protein in oocytes, it is important to collect the oocytes, in which mRNA is injected into the same hemispherical surface. In the present invention, it is possible to memorize information of a cellular area in the injection of the sample and to induce easily a correlation between the information and the subsequent cell reaction.

Further, the present invention provides a method for the automatic microinjection of sample into amphibian oocytes, comprising the following steps:
- using an apparatus having a tray for holding a plurality of the amphibian oocytes and an injection needle for injecting a sample into these amphibian oocytes,
- setting the depth of the injection needle relative to the tray or the amphibian oocytes to a first depth,
- injecting the sample into the first oocyte of the plurality of the amphibian oocytes using the injection needle at the first depth,
- automatically moving the relative position of the tray to the injection needle, and
- subsequently injecting the sample into the second oocyte of the plurality of the amphibian oocytes by using the injection needle at the first depth.

Furthermore, the present invention provides a method for the automatic microinjection of samples into amphibian oocytes, comprising the following steps:
- using an apparatus having a tray for holding a plurality of the amphibian oocytes and an injection needle for injecting a sample into the amphibian oocytes,
- injecting the sample into the first oocyte of the plurality of amphibian oocytes using the injection needle,
- moving the relative position of the tray relative to the injection needle,
- subsequently injecting the sample into the second oocyte of the plurality of amphibian oocytes using the injection needle,
- obtaining the condition of the oocyte in the injection of the sample as a visual information, and
- accumulating the visual information.

In addition to the above-described apparatus and method for injection of a sample into amphibian oocytes, the present invention further provides a plurality of the amphibian oocytes wherein the sample has been injected under substantially equal conditions regarding the injection depth of the sample.

The present invention also provides amphibian oocytes wherein the sample has further been injected under substantially the same conditions regarding the injection position of the sample.

In addition, the present invention provides the following methods.

A method for preparation of a group of amphibian oocytes injected with a sample, comprising
using an apparatus having a tray for holding a plurality of the amphibian oocytes and an injection needle for injecting a sample into the plurality of the amphibian oocytes,
moving the relative position of the injection needle to the tray,
injecting the sample into each of the plurality of the respective amphibian oocytes using the injection needle,
obtaining visual information of each of the amphibian oocytes in the injection, and
collecting a plurality of the oocytes into which the sample has been injected into the animal hemisphere of the oocyte, or a plurality of the oocytes in which the sample has been injected into the vegetal hemisphere of the oocyte amongst a plurality of amphibian oocytes based on the visual information.

The invention further relates to a method for selling or assigning a plurality of amphibian oocytes, comprising selling or assigning as a set a plurality of the amphibian oocytes, into which a sample has been injected under substantially equal conditions regarding the injection depth of the sample, and attaching the information on the injection of the sample into a plurality of the amphibian oocytes to the set.

In addition, the invention pertains to a method for selling or assigning a plurality of amphibian oocytes, comprising putting a plurality of amphibian oocytes into which a sample has been injected under substantially equal conditions regarding the injection depth of the sample into a vessel, and attaching a label on which information on the injection of the sample into a plurality of the amphibian oocytes is indicated or described to the vessel.

In this connection, the information on the injection of the sample into a plurality of amphibian oocytes relates to at least one of date and time of injection, the term for guarantee of the quality, the position where the sample has been injected, the depth at which the sample has been injected, and the probability of expression.

As a result, according to the present invention, oocytes having substantially identical conditions regarding the injection as well as the respective information can be obtained.

### Brief Description of the Drawings

- Fig. 1:: Construction of an apparatus of the present invention.
- Fig. 2:: Illustrative example of a tray used in the present invention.
- Fig. 3:: Illustrative example of a vessel used in the present invention.
- Fig. 4:: A correlation between the fluorescence of GFP injected into oocytes and the ligand response of histamine receptors expressed by co-injection.
- Fig. 5:: A correlation between the area of gene injection (animal hemisphere and vegetal hemisphere) and the ligand response.
- Fig. 6:: Illustration of the use of oocytes as histamine sensors.
- Fig. 7:: Illustration of a method of transferring oocytes after the injection of a sample.

### Explanation of symbols:

1: control unit, 2: auxiliary control unit, 3: monitor; 4: moving table for injection needle, 5: injection device, 6: injection needle, 7: CCD camera, 8: digital camera, 9: tray, 10: light source, 11: orthogonal moving table, 12: horizontal moving table, 13: oocytes, 14: physiological saline for amphibia, 21: vessel, 22: label, 23: cold insulator, 31: histamine receptor gene, 32: histamine receptor, 33: sample containing histamine, 34: histamine response (positive), 35: sample without histamine, 36: histamine response (negative), 41: syringe, 42: buffer solution for amphibia, 43: antibiotics, 44: outer vessel, 45: package.

### Description of the Preferred Embodiment

Referring to the drawings, the present invention will be explained in detail in the following:

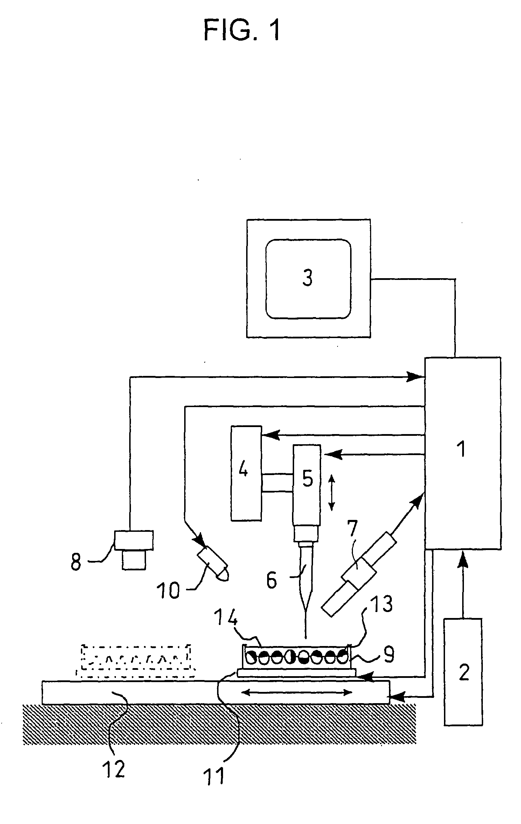

In Fig. 1, the principle of the present apparatus is shown.

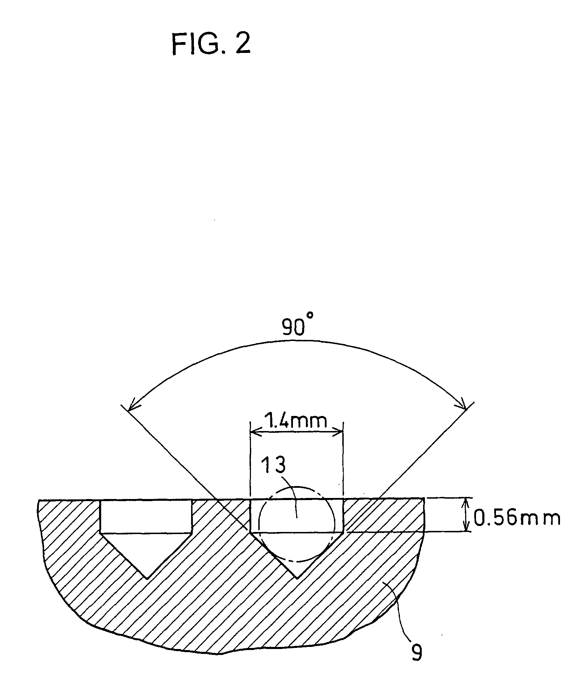

A tray, in which the oocytes are lined up and arranged, having, for example, arrangements of 12 wells in a horizontal direction and 8 wells in an orthogonal direction, in total 96 wells with a uniform depth and form, can be used, however, the number of wells is not limited to 96 wells. The amphibian oocytes have generally a heavier weight in the vegetal hemisphere than in the animal hemisphere. Consequently, by designing the diameter of the wells in the tray to be slightly larger than that of the oocytes in use, about 80 % of the oocytes in average can be maintained to keep their animal hemisphere upside, without changing the directions of oocytes arranged on the tray by rotating the cells, and the injection probability to the identical hemispherical surface can be increased without using any other special means. The wells for arranging oocytes are preferably of a cylindrical form, which has a circular flat basal plane with a constant cross section parallel to the base plane from the base plane to the open end aperture of the above well, or a form with a conical base. The diameter of the open end of the above wells should be larger than that of the amphibian oocytes in use. In addition, due to the above-mentioned reason, it is preferable to keep a space for rotating the oocytes in a well in which physiological saline is filled, and specifically the fact that a maximum diameter of the wells set to be 105 - 150 % of the diameter of the oocytes used is preferable, has been confirmed experimentally. For example, since the diameter of Xenopus oocytes is approximately 1.3 mm, it becomes possible to fix the oocytes in a specified direction without damaging the oocytes by designing the diameter of the well to be about 1.4 - 2 mm. The preferable form of a well in the present invention is, for example, as shown in Fig. 2, a conical shape with an angle of base at 90°, a diameter of 1.4 mm and a depth in the cylindrical part of 0.56 mm. Furthermore, by using a syringe in addition to the use of the above tray, it is possible to operate with an increased injection probability into the same surface side of different oocytes, or it is possible to operate so that the specified surface, for example, the vegetal hemispheres of the oocytes, are directed upward.

Examples of samples to be injected include, but are not limited to genes, pigments, proteins, peptides and drugs. In the example hereinbelow, oocytes, into which a histamine receptor cRNA is injected, will be described, but the injectable genes are not limited to cRNA and can be DNA, RNA and synthetic oligonucleotides. The needle for injecting the sample is preferably a pipette-like needle, but it is not limited to that form. In order to obtain cell information such as positional direction of oocytes, visual information through a digital camera 8 is applied. Further, means for detecting the contact of the injection needle 6 with the surface of the oocytes is examplified as visual information through a CCD camera 7. However, the means required for obtaining the information are not limited to the digital camera 8 and the CCD camera 7. For example, a sensor, which can detect changes of pressure, temperature, voltage, moisture or pH, may be mounted on the injection apparatus, and the surface of the oocytes can be detected based on this information.

The oocytes before injection of the sample are arranged in the wells on the tray 9, and physiological saline 14 is filled in the tray 9, then the tray is set on the orthogonal moving table 11 and the horizontal moving table 12. It is preferable to determine the position of the oocyte 13, into which the sample is injected from the injection needle 6, by controlling the movement of the orthogonal moving table 11 and the horizontal moving table 12 in the direction of the X-axis and the direction of the Y-axis with the control unit 1. As an alternative to the construction in Fig. 1, however, a construction can be applied with fixed tray 9 and with a movable injection needle 6.

When the tray 9 is located at the position indicated with the broken line, the oocyte may be photographed by the digital camera 8, and the image data may be transferred to the control unit 1, and information of quality and positional direction of the oocytes can be accumulated.

The horizontal moving table 12 and the orthogonal moving table 11 are operated by an indication of the control unit 1, and the center of the first oocyte 13, which is located in the defined position amongst the oocytes arranged on the tray 9, moves to the downward position under the position of the gene injection needle 6. At this point, the injection needle moving table 4 is operated by an indication of the control unit 1 for moving the injection needle in the direction of the Z-axis, the tip of the injection needle 6 mounted on the injection unit 5 moves downward to a position slightly distant from the surface of the oocyte, for example, close to the front by several Hundredths of mm. At this point, by observing the image taken by the CCD camera 7 on the monitor 3, indication is given from the auxiliary control unit 2, and the injection needle moving table 4 is operated to move in the descending direction slowly. The contact of the tip of the injection needle 6 with the surface of the oocyte 13 can be detected by visual information, pressure changes, temperature changes, electric changes, moisture changes, or pH changes; then the injection needle moving table 4 is stopped at this position. This position is a reference point for the subsequent gene injection operation. This point is made to be memorized in the control unit 1, and the following operation is performed. Namely, moving distance and depth of the injection needle for the vertical direction against a plane of the tray from the above reference point to the position of injecting sample are set, and the injection needle 6 is stuck at the setting depth to inject the previously fixed amount of the sample. For the injection of sample, a control for the Z-axis direction can be performed, for example, such that the injection needle moves downwardly to 0.2 mm from the contact point of the injection needle 6 on the surface of the oocyte 13. The optimum injection depth of the injection needle 6 into the oocyte is different depending on the type of sample to be injected and the object for injection and can be set freely. The sample can not diffuse into the oocyte, if the injection depth is too shallow, and if it is too deep, the probability for damaging the nucleus and the oocyte is increased. Consequently, it is preferable to inject a sample at almost constant depth from the standpoint of expression efficiency. For examle, in the case that mRNA is injected into the cytoplasm in order to express a protein, sticking the needle to a depth of 0.02-0.1 mm from the cell surface is preferable. On the other hand, in the case that DNA is injected into a nucleus in order to express a protein, sticking the needle to a depth of 0.05-0.2 mm from the cell surface is preferable on the animal hemisphere. However, since the form of the oocyte may be deformed at the injection, actually the sample is injected at a shallower position than the predetermined depth. The time for injecting the sample is controlled by setting the time for inserting the needle into the oocyte depending on the amount of sample to be injected. In order to improve the injection efficiency, a plurality of injection needles 6 can be used. In this case, the movement of the relative position between the injection needle and the tray by the indication from the driving unit of the apparatus in one-dimensional direction or two-dimensional direction may be sufficient.

Subsequently, the sample is automatically injected at the indicated time, rate and injection depth into the desired number of other oocytes in the tray 9 by automatic control. Further, since the size of the oocytes may have some deviation, a function for detecting the position of the surface at each time of injection can be applied. In addition, the information of the oocyte condition is memorized in the computer and can be put out on demand.

The system can be made so that the movement of the injection needle 6 and the oocyte 13 in the injection or the visual information of oocyte at the injection is memorized in the computer, and after termination of the injection operation, position and depth of the sample injection and the characteristics of the oocytes can be read out. In this case, the visual information of each oocyte is preferably correlated with the position on the tray by numbering the oocytes, for example.

The amphibian oocytes are known to comprise an animal hemisphere and a vegetal hemisphere, and each hemisphere has a different function. We have found that in case that mRNA was injected into the oocyte, the functional expression efficiency of protein was different depending on whether the mRNA was injected into the animal hemisphere or the vegetal hemisphere. In case of injecting histamine receptor mRNA, the expression efficiency is higher for injection into the animal hemisphere than the vegetal hemisphere; as a result oocytes with large ligand response can be obtained. On the other hand, in case that a protein, a fluorescent protein or a gene thereof and pigment are injected, information on color and light with higher sensitivity can be obtained by injection into the vegetal hemisphere. As described above, the injection of sample into the animal hemisphere can be made for about 80 % of oocytes on the tray by using the above tray. In case that oocytes wherein the sample is injected into a specific surface of the oocytes such as only the animal hemisphere or the vegetal hemisphere, are expected to be obtained, as described above, each oocyte is treated for arranging the specific hemisphere in upward direction by using the syringe before injection of sample. Alternatively, the area information for sample injection on the surface of oocytes is obtained by the detection means for the visual information or by a black and white discrimination sensor in the injection of sample, and as a result of the thus obtained information, the oocytes wherein the sample is injected into only the surface of interest directed for upward direction, can be collected. As a result, oocytes having substantially identical conditions regarding the injection position can be obtained. In the present invention, the "specific area" means positions including the animal hemisphere, the vegetal hemisphere or the equatorial area of the oocytes.

By using the apparatus having the above constitution, the sample can be injected at the specific area and depth of the amphibian oocytes, and the oocytes which have almost the same quality regarding the expression efficiency of the injected sample (injection efficiency), can be produced rapidly in mass production. Accordingly, the present invention provides a method for injecting a sample into amphibian oocytes at a specific position and at a specific depth by using the apparatus of the present invention.

It has been found that the sample injection efficiency can be improved by using the apparatus of the present invention as follws. Namely, in case of beginners, who have no experience for sample injection by manual operation, about 30 minutes are required for injecting samples into 25 cells, and the injection efficiency is about 30 % in case of the expression rate using a sample of a gene. On the contrary, using the apparatus of the present invention, the time for injecting samples into 25 cells is as low as only 3 minutes, and the injection efficiency reaches about 80 %. In case of sample injection performed by experts with manual operation, almost no shortage of time for injection is observed, but the injection efficiency can be improved up to 90 % by using the apparatus of the present invention as compared with the efficiency of 80 % in the manual operation.

Consequently, as a result of using the apparatus and method of the present invention, efficiencies of about 80-90 % can be achieved without being dependent upon the skill of operators, and the present invention permits to sell or assign a plurality of oocytes with controlled conditions of sample injection.

Therefore, in another aspect, the present invention provides amphibian oocytes with guaranteed injection of the sample at a specific position and at a specific depth.

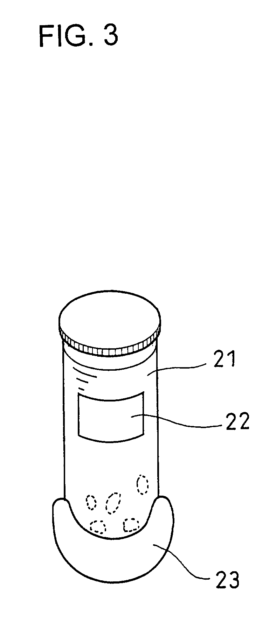

Further, oocytes, into which the sample has been injected at a specific area and depth, can be collected, sold or assigned. In the occasion of sale or assignment, a plurality of oocytes are packaged, and a label 22, in which information on the type of sample, the injection date, the term for guarantee of quality, the setting conditions such as the position and the depth of sample injection, and the guaranteed injection efficiency is described, can be attached (Fig. 3).

According to the present invention, the oocytes can be sold or assigned with the information on the specific depth and area of the injection, and on the expression of protein encoded by the injected gene. In the event of selling or assigning the guaranteed oocytes regarding efficiency of sample injection or efficiency of expression, the efficiency of sample injection can be guaranteed, for example, by co-injecting a sample with protein containing pigment or chromophore, fluorescent protein, or gene encoding these proteins, counting the number of oocytes emitting color or fluorescence, and indicating the ratio as an indicator of efficiency of sample injection. Although the coinjection can be performed in the mixed form, in case that both the sample and a protein for detection to be coinjected are injected in the form of a gene, wherein a gene coding a fused protein can be used.

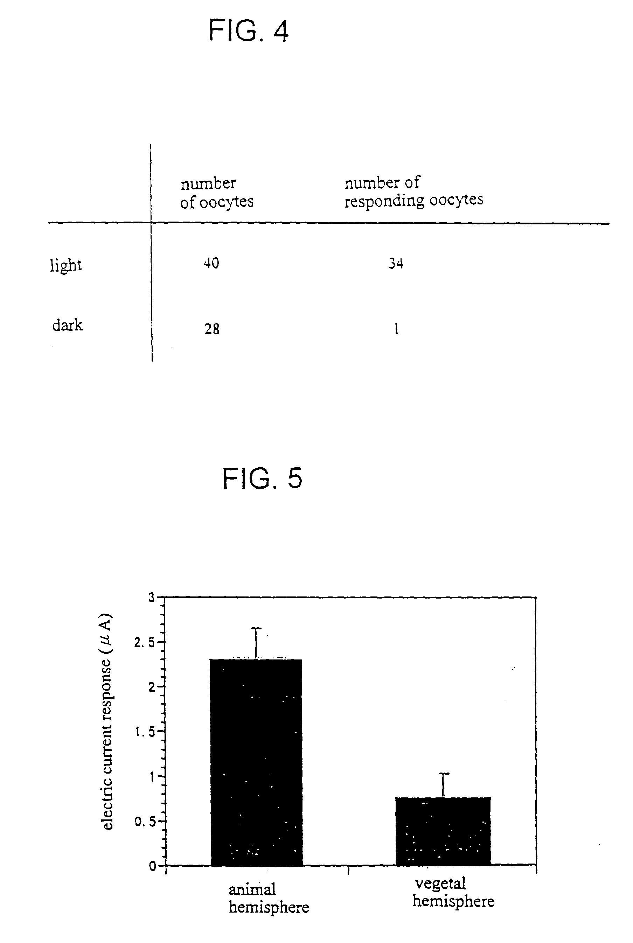

Referring to Fig. 4, an example for calculating the efficiency of sample injection or efficiency of expression by using fluorescence from co-injected fluorescent protein as an indicator, will be explained. In the present example, the case that the rate of injection of histamine receptor gene is determined by using the expression of the green fluorescent protein derived from Aequorea forbesiana (GFP) as an indicator, is explained. However, the injected samples are not limited to genes. Further, substances used as indicators of the efficiency of sample injection are not limited to GFP.

A mixture of histamine receptor gene and green fluorescent protein gene is injected into the oocyte by using an apparatus of the above construction or realizing the above principles. 24 hours after the gene injection manipulation, light of a wavelength of 488 nm is irradiated to the oocytes, and then the expressed green fluorescent protein emits fluorescence of 507 nm. Oocytes with emission of fluorescence of 507 nm are classified into "light", and oocytes without emitted fluorescence are classified into "dark". Fig. 4 shows that these oocytes are stimulated with histamine and are classified by the presence or absence of response. As shown in Fig. 4, in the "light" oocytes, 85 % of oocytes (34 out of 40 cells) respond to histamine. Namely, histamine receptor gene can be injected in more than 85 % of the oocytes. On the contrary, in the "dark" oocytes, 90 % or more of oocytes cannot respond to histamine (27 out of 28 cells). Namely, the histamine receptor gene can not be injected in 90 % or more of oocytes. Accordingly, it is demonstrated that the frequency of injection of the histamine receptor gene is high in oocytes expressing green fluorescent protein.

As clearly demonstrated by the above fact, when the objective sample is co-injected with the fluorescent protein, the efficiency of injection of the objective sample can be calculated based on the existence of fluorescence as the indicator; as a result, sale or assignment of oocytes with guaranteed efficiency of sample injection is possible.

Next, means for production, sale or assignment of oocytes obtained by the present invention for a specific use, which is exemplified by using oocytes injected with human histamine receptor cRNA, will be described. However, the genes for use for injection are not limited to cRNA, and DNA, RNA and oligonucleotides can be used.

The histamine receptor cRNA is injected into oocytes by using the apparatus and the method of the present invention. In this case, when means for obtaining visual information such as sensor for discriminating black and white color or a CCD camera/digital camera is used, the oocytes into which cRNA is injected in the animal hemisphere, and the oocytes into which cRNA is injected in the vegetal hemisphere can be differentiated.

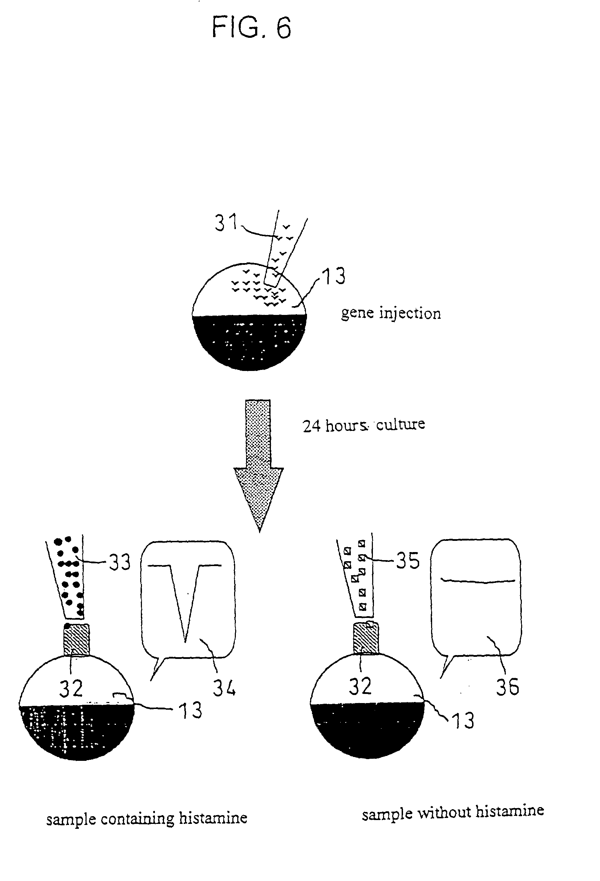

After gene injection, the histamine receptor is expressed in the oocytes within 24 hours. After passing 24 hours from histamine receptor gene injection, the membrane potential of the oocytes, in which histamine receptor may be expressed, is held at -60 mV by the two electrodes voltage clamp method. Under such conditions, addition of sample containing histamine to the oocytes results in the interaction between the histamine in the sample and the histamine receptors, and the signal transduction system in the oocyte is activated to generate an ionic current, then the electric response of the oocytes to histamine can be shown. The oocytes after 24 hours are stimulated with 1 mM histamine and its electric current response is measured. Fig. 5 shows a comparison of the electric current response of the oocytes into which gene is injected in the animal hemisphere and in the vegetal hemisphere. As shown in Fig. 5, differences in the electric current response to histamine depending on the injection site of the gene are observed.

Namely, oocytes with good ligand response can be obtained in the case that the gene is injected in the animal hemisphere rather than in the case that the gene is injected in the vegetal hemisphere.

Consequently, for example, the group of oocytes into which histamine receptor cRNA has been injected only in the animal hemisphere, can be used as good sensors with high sensitivity for histamine. Further, according to the present invention, sale or assignment of oocytes, into which the sample has been injected at a specific position and depth, for the purpose of using them as sensors can be made. As easily recognized by the person skilled in the art, examples of samples to be injected for use of oocytes obtained by the present invention as sensors, are also genes encoding receptors for any ligands other than for histamine, antibodies having reactivity for specific antigens, and glycoproteins having specific sugar chain, but are not limited to the above examples.

The histamine receptor gene 31 is injected into the oocyte 13 by using the apparatus assembled with the above constitution or using the above principles. In Fig. 6, the injection into the vegetal hemisphere is shown. After injection of the histamine receptor gene, the histamine receptor 32 is expressed in the oocytes 13 within 24 hours. In the same way as described above, after passing 24 hours from the histamine receptor gene injection, the membrane potential of the oocytes, in which histamine receptor 32 may be expressed, is held at -60 mV by the two electrodes voltage clamp method. Under such conditions, the addition of a sample 33 containing histamine to the oocytes 13 results in the interaction between the histamine in the sample and the histamine receptors, and the signal transduction system in the oocyte is activated to generate an ionic current; then the electric response 34 of the oocyte 13 to histamine can be shown. In case that a sample 35 without histamine is added, wherein no substance exists which interacts with the receptor, the oocyte 13 cannot respond to histamine 36.

The oocyte expressed histamine receptor response to histamine containing samples can be used as indicator. Since the mass production of oocytes having identical conditions for injection of the sample is possible by using the apparatus for sample injection of the present invention, the amphibian oocytes can be used for screening of the ligand or antigen reacting with receptor or antibody. The screening can be performed by using a plurality of oocytes, in which a sample such as a gene has been injected under substantially equal conditions and protein or other substances are expressed, and comparing the results of reactions of oocytes with different ligands.

Further, in another use according to the present invention, for example, the expressed protein can be extracted by disintegrating the oocytes in which protein is expressed. The protein and other products can be effectively extracted, for example, by controlling the conditions for injection with using an apparatus of the present invention, and by using oocytes, into which sample has been injected into the animal hemisphere.

A method for transporting oocytes according to the present invention, to which a sample has been injected, will be described referring to Fig. 6. In case of sale and transport of the above oocytes, the oocytes 13 are put into a vessel 21, into which a solution of buffer conventionally used for amphibian oocytes with dissolved antibiotics such as gentamicin sulfate, penicillin and streptomycin is filled. The vessel 21 is packaged using packaging material such as styrene foam, and is preferably transported by maintaining the temperature at 4 - 25 °C, more preferably at 18 - 22 °C, by using a cold insulator 23 while avoiding shock, as shown in Fig. 3.

The composition of the solution is not limited, and the composition given below can be preferably used. The solution pH is adjusted to 7.5.

| | |
|---|---|
| NaCl | 96 mM |
| KCl | 2 mM |
| CaCl₂ | 1.8 mM |
| MgCl₂ | 1 mM |
| HEPES | 5 mM |
| Gentamicin sulfate | 50 mg/ml |
| sodium pyruvate | 2.5 mM |
| Penicillin | 10 U/ml |
| Streptomycin | 10 mg/ml |

Examples of preferable vessels for sale and transport are not limited to the above construction. Preferred vessels are those in which the oocytes can move relatively freely inside with the cap freely opened and closed, and the above solution is preferably filled to about 95 % of the vessel volume. For example, in case of a conical tube of 50 ml, preferably about 100 - 200 oocytes, more preferably about 130 - 180 oocytes, are contained. This ratio corresponds to about 0.3 - 0.5 ml/oocyte, but is varied depending on the type of oocytes and the type of vessels.

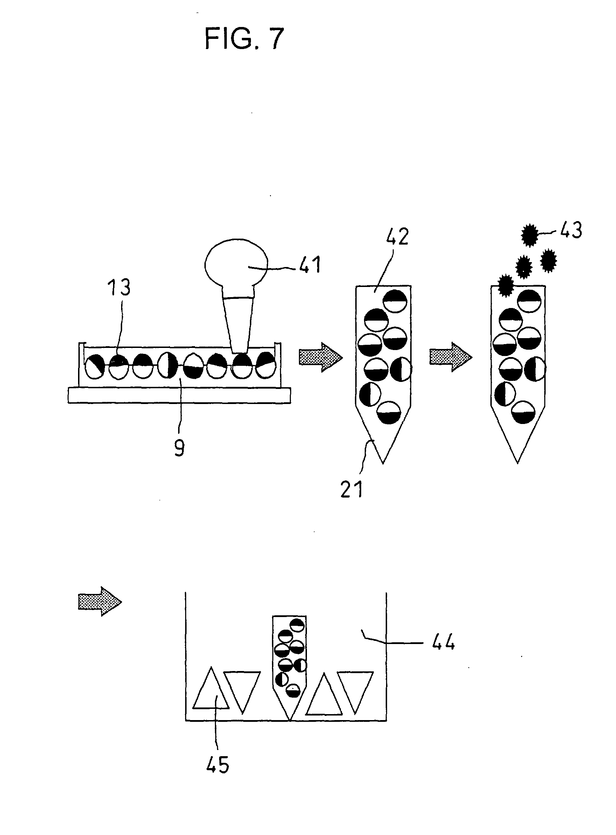

As shown in Fig. 7, the oocyte 13, into which a sample has been injected, is recovered from the tray 9 by using a syringe 41. In this time, based on the recorded information, any one of oocytes, wherein the sample has been injected into the animal hemisphere or the vegetal hemisphere, can be recovered. The recovered oocytes are transferred into the vessel 21. Buffer solution for amphibia 42 is filled in the vessel 21, and the solution is exchanged several times. After the exchange, buffer solution for amphibia 42 is filled in the vessel 21 again, and a preferable amount of antibiotics 43 is added thereto. The cover of tube is closed, and the tube is put into the outer case 44 with cold insulator. The outer case 44 is filled with packaging material 45, fixing the vessel 21 and transported to the customer by conventional transporting means.

By this method, the vessel can be transported to the customer without damaging the function of oocytes to which the sample has been injected.

In the occasion of sale or assignment, information including date of injection, place of injection of sample, conditions for injection such as depth, recovery rate and term for guarantee of quality, is provided. For that purpose, for example, a paper describing such information or a label 22 may be attached to the vessel 21 containing the oocytes (Fig. 3).

In case of gene injection, about 24 hours are required for expression, and the lifespan of the oocytes is about 7 days after injection, preferably for safety about 5 days. Consequently it is preferable to describe date and time of injection and, corresponding to that, a description indicating that "best use before X X (day - month)" in order to clearly indicate the effective term after injection.

As for the injection site of the oocyte, the sample can be injected into the animal hemisphere in about 80 % of oocytes by using the above tray, and it is also possible to improve the expression efficiency based on the accumulated information in the apparatus as well as by using co-expression with use of the present apparatus.

### Effect of the Invention

According to the present invention, a sample can be injected into oocytes of amphibia such as frogs with a constant depth precisely, and mass production of oocytes having an identical quality such as efficiency of injection can be rapidly performed. The quality of an oocyte or the area of needle injection can be recorded as information.

Further, the oocytes obtained by the method of the present invention and into which the sample has been injected in the specified position and depth, can be recovered, and the efficiency of injection of sample is guaranteed for the purpose of sale or assignment. In addition, selling or assigning of the oocytes can be made by specifying the use depending on the type of the injected sample.

## Claims

1. Apparatus or system for the microinjection of samples into amphibian oocytes, comprising
- a tray (9) for holding a plurality of the amphibian oocytes (13),
- an injection needle (6) for injecting the sample into the amphibian oocytes (13),
- driving means (4, 11, 12) for moving the relative position of the tray (9) to the injection needle (6),
and
- controlling means (1, 2) for controlling the movement by setting the depth of the injection needle (6) relative to the tray (9) or the amphibian oocytes (13) in the sample injection, and for injecting the sample into a plurality of the amphibian oocytes (13) at that depth.

2. Apparatus according to claim 1, comprising one or more of the following features:
- the driving means (4, 11, 12) are designed to drive the tray (9) relative to the injection needle (6) in a three-dimensional manner;
- it further comprises information obtaining means (7, 8) for obtaining a visual information of the amphibian oocytes (13) in the injection of the samples;
- the information obtaining means (7, 8) for obtaining visual information of the amphibian oocytes (13) are a camera;
- it further comprises means for connecting each visual information of the amphibian oocytes collected by the information obtaining means (7) for the visual information with the position of each oocyte on the tray (9);
- it further comprises memorizing means (1, 2) for memorizing the visual information;
- the tray (9) has a plurality of wells for holding the plurality of amphibian oocytes (13);
- the wells have a cylindrical structure with a flat base or with a conical base having a maximum diameter of 1.4 to 2 mm;
- the wells have a cylindrical structure with a flat base or with a conical base having a maximum diameter of 105 to 150 % of the diameter of the amphibian oocytes (13);
- the position of the surface of oocytes on the tray (9) is detected by at least one of visual information, pressure change, temperature change, electrical change, moisture change or pH change.

3. Apparatus or system according to claim 1 or 2, comprising information obtaining means (7, 8) for obtaining a visual information of the amphibian oocytes (13) in the injection of the samples, and memorizing means (1, 2) for accumulating the information, and means for injecting the sample into the amphibian oocytes (13).

4. Method for the microinjection of samples into amphibian oocytes (13), comprising the following steps:
- using an apparatus having a tray (9) for holding a plurality of the amphibian oocytes (13) and an injection needle (6) for injecting samples into the amphibian oocytes (13),
- setting the depth of the injection needle (6) relative to the tray (9) or the amphibian oocytes (13) to a first depth value;
- injecting the sample into the first oocyte of the plurality of amphibian oocytes (13) using the injection needle (6) at the first depth value,
- automatically moving the relative position of the tray (9) to the injection neelde (6), and
- subsequently injecting the sample into the second oocyte of the plurality of amphibian oocytes (13) by using the injection needle (6) at the first depth value.

5. Method for microinjection of samples into amphibian oocytes (13), comprising the following steps:
- using an apparatus having a tray (9) for holding a plurality of the amphibian oocytes (13) and an injection needle (6) for injecting samples into the amphibian oocytes (13),
- injecting the sample into the first oocyte of the plurality of amphibian oocytes (13) using the injection needle (6),
- moving the relative position of the tray (9) relative to the injection needle (6),
- subsequently injecting the sample into the second oocyte of the plurality of amphibian oocytes (13) using the injection needle (6),
- obtaining the condition of the oocyte in the injection of the sample as a visual information, and
- accumulating the visual information.

6. Method according to claim 4 or 5, comprising one or more of the following features:
- the sample is gene or protein;
- the sample contains a fluorescent substance;
- the amount of sample injected into the first oocyte is controlled, and the sample is injected into the second oocyte in a substantially equal amount;
- the samples are injected under substantially equal conditions, particularly regarding the injection depth;
- the samples are injected under substantially equal conditions regarding the injection area;
- the amount of injection of the sample is kept substantially constant;
- genes, pigments, proteins, peptides and/or drugs are injected;
- cRNA, DNA, RNA and/or synthetic oligonucleotides are injected.

7. Process for the preparation of amphibian oocytes (13) injected with a sample, comprising the following steps:
- using an apparatus having a tray (9) for holding a plurality of the amphibian oocytes (13) and an injection needle (6) for injecting a sample into the amphibian oocytes (13),
- moving the relative position of the injection needle (6) to the tray (9),
- injecting the sample into each of the amphibian oocytes (13) using the injection needle (6),
- obtaining visual information of each of the amphibian oocytes (13) in the injection, and
- collecting a plurality of the oocytes in which the sample is injected into the animal hemisphere of the oocyte amongst the plurality of amphibian oocytes (13) based on the visual information,
and/or
- collecting a plurality of the oocytes in which the sample is injected into the vegetal hemisphere of the oocyte amongst the plurality of amphibian oocytes (13), based on the visual information.

8. The process according to claim 7, comprising the following steps:
- setting the depth of the injection needle (6) relative to the amphibian oocytes (13) or the tray (9) to a first depth prior to the injection, and
- injecting the sample into a plurality of the amphibian oocytes (13) at the first depth.

9. Method for selling or assigning a plurality of amphibian oocytes, comprising selling or assigning as a set of a plurality of the amphibian oocytes, into which a sample is injected under substantially the same conditions regarding the injection depth.

10. Method according to claim 9, comprising one or more of the following features:
- attaching an information on the sample injection into a plurality of the amphibian oocytes to the set;
- putting a plurality of the amphibian oocytes into which the sample is injected under substantially the same conditions regarding the injection depth into a vessel (21), and
- attaching to the vessel (21) a label (22) comprising information on the sample injection into a plurality of the amphibian oocytes (13);
- the vessel (21) is maintained at a temperature not lower than 18 °C and not higher than 25 °C;
- the information on the sample injection into a plurality of the amphibian oocytes (13) relates to at least one of the date and time of the injection, the term for guarantee of quality, the area where the sample is injected, the depth at which the sample is injected, and the probability of expression.

11. Method to use a plurality of amphibian oocytes (13) into which a gene or a protein is injected at an approximately constant position and depth, as a sensor for screening.

12. A plurality of amphibian oocytes (13) into which samples have been injected under substantially equal conditions regarding the injection depth.

13. The plurality of amphibian oocytes (13) according to claim 12, wherein the sample has further been injected under substantially equal conditions regarding the injection area.

14. The plurality of amphibian oocytes according to claim 12 or 13, wherein the amount of injection of the sample is also substantially constant.

15. A vessel containing amphibian oocytes (13) into which a sample has been injected under substantially the same conditions regarding the injection depth, and having information on date and time of injection and information on the expiration date for use attached thereto.

16. The vessel according to claim 15, containing amphibian oocytes (13) into which a sample has been injected under substantially equal conditions regarding the injection area and/or the amount of injection.
